Europäisches Patentamt

(19) European Patent Office

Office européen des brevets

(11) Publication number: **0 191 995**

A1

(12) **EUROPEAN PATENT APPLICATION**

(21) Application number: 85309528.9

(22) Date of filing: 30.12.85

(51) Int. Cl.⁴: **C 07 C 45/41**
C 07 C 47/52, C 07 C 47/02
B 01 J 23/34, B 01 J 23/76

(30) Priority: 22.01.85 US 693245
22.01.85 US 693246

(43) Date of publication of application:
27.08.86 Bulletin 86/35

(84) Designated Contracting States:
AT BE CH DE FR GB IT LI LU NL SE

(71) Applicant: CHEM SYSTEMS INC.
303 South Broadway
Tarrytown New York 10591(US)

(72) Inventor: Gelbein, Abraham P.
45 Headley Road Morristown
Morris New Jersey 07960(US)

(72) Inventor: Hansen, Robert
50 Laurel Place West Caldwell
Essex New Jersey 07006(US)

(72) Inventor: Holy, Norman L.
4 Lee Drive
Maple Glen, Pennsylvania 19002(US)

(74) Representative: Harrison, Michael Robert et al,
Urquhart-Dykes & Lord 5th Floor Tower House Merrion
Way
Leeds, LS2 8PA(GB)

(54) Hydrogenation catalysts and their use.

(57) A process for the hydrogenation of carboxylic acids and esters to form the corresponding aldehydes involves reaction with a reducing gas at a temperature from 300 to 500°C and a pressure from 200 mm Hg to 100 atm. in the presence of a manganese dioxide catalyst supported on an activated alumina or an yttrium oxide catalyst which has been treated with copper, the catalyst comprising from 1 to 15 wt.% of copper and from 99 to 85 wt.% of yttrium, on an elemental basis. The catalyst is also claimed.

EP 0 191 995 A1

Croydon Printing Company Ltd.

BJL:WP
12/2/85
7092-1218FF

## HYDROGENATION CATALYSTS AND THEIR USE

### Background of the Invention

The catalytic vapor-phase hydrogenation of carboxylic acids and esters to form the corresponding aldehydes is well known. Hydrogenation processes wherein the carboxylic acids and esters are free of hydrogen on the alpha-carbon, such as benzoic acid and methyl benzoate, are taught in U.S. Patent 4,328,373, issued on May 4, 1982 to Dow Chemical Company. The reaction is performed in the presence of metal oxide catalysts such as oxides of yttrium, zirconium, cerium, praseodymium, thorium and uranium supported on alpha-alumina. The first two oxides are most preferred. This work is also described in King et al., "An In Situ Study of Methyl Benzoate and Benzoic Acid Reduction on Yttrium Oxide by Infrared Spectroscopic Flow Reactor," Journal of Catalysis 76, 274-284 (1982).

U.S. Patent 4,093,661, issued June 6, 1978 to Union Carbide Corporation, shows the vapor phase disproportionation of lower alkanoate esters of alcohols in the vapor phase to produce aldehydes and ketones over metal oxide catalysts. The preferred catalysts include nickel oxide, zinc oxide, and chromium oxide. The use of other metal oxides, namely, oxides of copper, titanium, vanadium, manganese, iron and cobalt, are also disclosed. It is further taught that the catalyst can be supported on an inert catalyst support such as alumina, silica and carbon.

In U.S. Patent 2,018,350, issued on October 22, 1935 to General Aniline Works, Inc., aldehydes are formed from dicarboxylic acids or their anhydrides with reducing gas in the presence of catalysts such as chromium, iron, copper, manganese, cobalt or their oxides, either alone or mixed with each other. Mixtures of such catalysts with other elements such as lead, cerium, uranium or zinc or oxides of these elements are also generally disclosed. The catalysts include granules of pumice impregnated with iron salts and reduced with hydrogen to prepare benzaldehyde from phthalic anhydride. In addition, a catalyst prepared by reducing fragments of ferric oxide activated with chromium compounds is also described. Other catalysts include copper and iron deposited on granular pumice, a reduced mixture of lead oxide, chromium oxide, and iron oxide, and reduced zinc chromate.

## Summary of the Invention

The first embodiment of this invention relates to the catalytic vapor-phase hydrogenation of carboxylic acids and esters to form corresponding aldehydes with a manganese catalyst supported on activated alumina. Such alumina include gamma, beta, and eta alumina. The use of the activated alumina support substantially enhances the catalytic activity. These supports are characterized by high surface area, i.e., 2 to 1000 $m^2$/g., preferably from 100 to 500 $m^2$/g., and high pore volume, i.e., 0.3 to 1.0 cc/g.

The second embodiment of this invention relates to the aforesaid reactions with an yttrium oxide catalyst treated with copper. The copper treatment of the catalyst has been found to substantially enhance its activity.

The above catalysts are particularly useful for converting carboxylic acids and their esters free of hydrogen on their alpha carbon to aldehydes. They exhibit substantially enhanced activity and selectivity over the prior art. An example of the reaction for which the catalysts of the invention may be used is the conversion of benzoic acid and methyl benzoate to benzaldehyde. Acids having hydrogen on alpha carbons such as acetic acid and butyric acid are not selectively converted with the activated alumina supported manganese catalyst of the invention.

## Detailed Description of the Invention

Carboxylic acids and esters thereof which can be converted to aldehydes in accordance with the instant invention may be either mono- or dicarboxylic. The esters include aryl and lower alkyl esters of such carboxylic acids. Both aliphatic carboxylic acids and aromatic carboxylic acids may be reacted.

Aromatic carboxylic acids and esters include benzoic, phthalic, isophthalic and terephthalic, as well as alkyl, alkoxy and halo-substituted benzoic acids and esters such as toluic, ethylbenzoic, fluorobenzoic, chlorobenzoic, bromobenzoic and methoxybenzoic. Preferably, the alkyl-substitution would contain from 1 to 6 carbon atoms. Generally, from 1 to 5 alkyl substitutions may be present. Other materials which may be converted include naphthoic, biphenylcarboxylic, anthranoic, trimethylacetic and trifluoroacetic acids and esters.

The supported manganese catalyst may be readily prepared by immersing the support in an aqueous solution of a manganese salt. The manganese salt solution loaded support is then heated at a temperature of from 200 to 500°C under oxidizing conditions to form supported manganese dioxide. Generally, from 2 to 20 wt. % of the catalyst is added to the support.

The yttrium oxide catalyst may be readily prepared as described in the aforementioned U.S. Patent 4,328,373. Generally, a salt of the yttrium compound is heated at a temperature of from 200 to 500°C under oxidizing conditions to form the corresponding metal oxide. The catalyst may be in the form of pellets or deposited on an inert support material, as for example alumina, silicon carbide, silica, and silica-alumina. Alpha-alumina is preferred.

Generally, from 2 to 20 wt. % of the catalyst is added to the support.

The copper activator, which forms the essence of the catalyst of the invention, may be formed at the same time as the yttrium oxide by means of the reduction of the yttrium and the copper salt simultaneously or the copper oxide may be intimately mixed with the yttrium oxide catalyst. The catalyst comprises from 1 to 15 wt. % copper and from 99 to 85 wt. % yttrium on an elemental basis, preferably from 5 to 8 wt. % of the copper and from 95 to 92 wt. % of the yttrium.

Prior to the reduction of the carboxylic acid or carboxylic acid ester, it is preferable to reduce the catalyst to its metallic form in situ under the reaction conditions.

The hydrogenation reaction may be carried out in a batch or a continuous flow system, as will be readily understood by those skilled in the art. The temperature of the reaction may range from 350° to 500°C, preferably from 400 to 450°C. Pressures of from 200 mm Hg up to a high as 100 atm. may be used, though 1 to 5 atm. is preferred.

The molar ratio of the hydrogen to the carboxylic acid or the carboxylic acid ester may range broadly from 1:1 to 1,000:1, preferably from 20:1 to 200:1. The optimum conditions of temperature, pressure and hydrogen concentration may be readily determined by those skilled in the art, depending on the particular reactant being employed.

The rate of flow of the reactants over the catalyst may range from 0.05 to 2.0 kg acid per kg catalyst/hr., preferably from 0.1 to 1.0 kg acid per kg catalyst/hr.

The following examples further illustrate the instant invention:

Example 1

Catalysts were prepared by immersing various supports in an aqueous solution of manganese nitrate. The solution and support were heated so as to reflux the solution at about 100°C for two hours. After cooling to room temperature, the excess solution was decanted and the impregnated support was dried at about 120°C and calcined at 450°C to produce the manganese dioxide. A catalyst loading of from about 10 to 20 wt. % of the oxide is easily achieved by this procedure. The catalysts in oxide form were reduced in the reactor at reaction conditions prior to the addition of the feed.

In the reactor system, the organic reactant was first placed in a constant temperature vaporizer. The hydrogen reducing gas was preheated and sparged into a reservoir of the heated reactants. From vapor pressure data, the desired concentration of organic reactant in hydrogen was determined. The hydrogen stream, saturated with organic reactant, was immediately heated to the reaction temperature and passed over the catalysts. The products exiting from the reactor were condensed and collected for analysis.

The following table shows the comparative results obtained. Run No. 1 demonstrates the use of the manganese dioxide catalyst supported on 1/8" gamma-alumina in accordance with the invention. Run Nos. 2 and 3 show comparative runs using the same catalyst supported on 8-12 mesh silica gel and 1/8" alpha-alumina spheres, respectively. The residence times were measured at reaction conditions on an empty tube basis.

Table i

| Run # | 1 | 2 | 3 |
|---|---|---|---|
| Reactor Volume (cc) | 8.2 | 8.0 | 9.0 |
| Catalyst Charge (gram) | 5.00 | 3.55 | 7.35 |
| Catalyst, % $MnO_2$ | 15.9 | 18.6 | 14 |
| Support Pore Volume, cc/g. | 0.92 | 1.0 | 0.8 |
| Support Surface Area, $m^2/g.$ | 194 | 300 | <1 |
| Residence Time (seconds) | 0.62 | 0.79 | 0.60 |
| F/W (Kg Feed/Kg Catalyst-Hr.) | 0.426 | 0.47 | 0.334 |
| Reactor Temp. °C | 433 | 435 | 434 |
| Feed: Mole % Benzoic Acid in $H_2$ | 2.1 | 1.80 | 1.8 |
| % Conversion of Benzoic Acid | 91 | 63 | 72.1 |
| % Molar Selectivity to: - | | | |
|    Benzaldehyde | 94.8 | 55 | 72.5 |
|    Benzene | 3.6 | 44 | 27 |
|    Toluene | 0.9 | 0.5 | 0.3 |
|    Benzyl Alcohol | 0.7 | 0.5 | 0.2 |

It will be noted in Run No. 1 that the conversion of benzoic acid was considerably higher than in Run Nos. 2 and 3. That the support would have this marked effect is quite unexpected. Even more surprisingly, by using the gamma-alumina supported manganese catalyst of the invention a substantially higher molar selectivity to benzaldehyde was obtained. By-product formation in Run No. 1 is manyfold less than in Run Nos. 2 and 3.

Example 2

A procedure described in Run No. 1 is followed, except that the feed is methyl benzoate. In a 2 hour run at 430°C, and with a feed stream containing 2.0% methyl benzoate, a conversion of 92% of the methyl benzoate is noted. The selectivity to benzaldehyde is 83%.

Example 3

The catalyst described in Run No. 1 is employed in a 5 hour run with p-methylbenzoic acid at 425°C. The conversion is 91% and the selectivity to p-methylbenzaldehyde is 86%.

Example 4

The catalyst described in Run No. 1 is employed in a 10 hour run with p-tert-butylbenzoic acid at 430°C. The conversion is 95% with a selectivity to p-tert-butylbenzaldehyde of 86%.

Example 5

The catalyst described in Run No. 1 is employed in a 4 hour run with 3,5-dimethylbenzoic acid at 435°C. The conversion is 87% at a 2.7% feed, with a selectivity to 3,5-dimethylbenzaldehyde of 83%.

Example 6

The catalyst described in Run No. 1 is employed in a 5 hour run with methyl p-methylbenzoate at 430°C. The conversion is 85%, and the selectivity to p-methylbenzaldehyde 85%.

### Example 7

The catalyst described in Run No. 1 is employed in a 5 hour run with methyl p-methoxybenzoate at 420°C. The conversion is 87%. The selectivity to p-methoxybenzaldehyde and to anisole is 12% and 75%, respectively.

### Example 8

The catalyst described in Run No. 1 is used in a 2 hour test with p-fluorobenzoic acid at 430°C. The conversion is 75%, and the selectivity to p-fluorobenzaldehyde 89%.

### Example 9

The catalyst described in Run No. 1 is used in a 4 hour test with 4-phenylbenzoic acid at 433°C. The conversion is 92%, and the selectivity to p-phenylbenzaldehyde 84%.

### Example 10

The catalyst described in Run No. 1 is used in a 4 hour test of trimethylacetic acid at 423°C. The conversion is 78%, and the selectivity to trimethylacetaldehyde 72%.

### Example 11

The catalyst described in Run No. 1 is used in a 10 hour test with dimethylterephthalate at 435°C. The conversion is 89%, and the selectivity to terephthalaldehyde 79%.

Example 12

Catalysts were prepared by immersing an alpha-alumina support in an aqueous solution of the catalyst components in the form of nitrates. The solution and support were heated so as to reflux the solution at about 100°C for two hours. After cooling to room temperature, the excess solution was decanted and the impregnated support was dried at about 120°C and calcined at 450°C to produce the metal oxide. A catalyst loading of from about 10 to 20 wt. % of the metal oxide is easily achieved by this procedure. The catalysts in oxide form were reduced in the reactor at reaction conditions prior to the addition of the feed.

In the reactor system, the organic reactant was first placed in a constant temperature vaporizer. The hydrogen reducing gas was preheated and sparged into a reservoir of the heated reactants. From vapor pressure data, the desired concentration of organic reactant in hydrogen was determined. The hydrogen stream, saturated with organic reactant, was immediately heated to the reaction temperature and passed over the catalysts. The products exiting from the reactor were condensed and collected for analysis.

The following table shows the comparative results obtained. Run No. 1 demonstrates the use of an yttrium oxide catalyst in accordance with the prior art. Run No. 2 shows the copper-activated catalyst of the instant invention. In both cases, the catalysts were supported on an alpha-alumina support in the form of 1/8" spheres. In Run No. 2, the percents copper and yttrium were 9% and 91%, respectively, on an elemental basis.

Table 2

| Run # | 1 | 2 |
|---|---|---|
| Reactor Volume (cc) | 17.0 | 17.0 |
| Catalyst Charge (gram) | 14.72 | 14.58 |
| Catalyst | 12.9% $Y_2O_3$ | 13.3% $Y_2O_3$/CuO |
| Residence Time (seconds) | 1.9 | 1.3 |
| F/W (Kg Feed/Kg Catalyst-Hr.) | 0.033 | 0.186 |
| Reactor Temp. °C | 430 | 428 |
| Feed: Mole % Benzoic Acid in $H_2$ | 1.8 | 2.3 |
| % Conversion of Benzoic Acid | 87.5 | 88 |
| % Molar Selectivity to: | | |
| Benzaldehyde | 92.9 | 94.2 |
| Benzene | 7.1 | 5.8 |
| Toluene | – | – |
| Benzyl Alcohol | – | – |

It will be noted in the above runs that the flow rate in Run No. 2 (using the copper-activated catalyst of the invention) is over twice that of Run No. 1. Despite this, the conversion to benzoic acid in Run No. 2 is comparable and a somewhat higher molar selectivity to benzaldehyde was obtained. This is particularly surprising and clearly shows that the copper-activated catalyst of the invention is far more active than the yttrium catalyst per se.

Example 13

A procedure described in Run No. 2 of Example 12 is followed, except that the feed is methyl benzoate. In a 2 hour run at 430°C, and with a feed stream containing 2.0% methyl benzoate, a conversion of 92% of the methyl benzoate is noted. The selectivity to benzaldehyde is 93%.

Example 14

The catalyst described in Run No. 2 of Example 12 is employed in a 5 hour run with p-methylbenzoic acid at 425°C. The conversion is 91% and the selectivity to p-methylbenzaldehyde is 94%.

Example 15

The catalyst described in Run No. 2 of Example 12 is employed in a 10 hour run with p-tert-butylbenzoic acid at 430°C. The conversion is 95% with a selectivity to p-tert-butylbenzaldehyde of 94%.

Example 16

The catalyst described in Run No. 2 of Example 12 is employed in a 4 hour run with 3,5-dimethylbenzoic acid at 435°C. The conversion is 87% at a 2.7% feed, with a selec-ivity to 3,5-dimethylbenzaldehyde of 93%.

Example 17

The catalyst described in Run No. 2 of Example 12 is employed in a 5 hour run with methyl p-methylbenzoate at 430°C. The conversion is 85%, and the selectivity to p-methylbenzaldehyde 95%.

Example 18

The catalyst described in Run No. 2 of Example 12 is employed in a 5 hour run with methyl p-methoxybenzoate at 420°C. The conversion is 87%. The selectivity to p-methoxybenzaldehyde and to anisole is 12% and 75%, respectively.

Example 19

The catalyst described in Run No. 2 of Example 12 is used in a 2 hour test with p-fluorobenzoic acid at 430°C. The conversion is 75%, and the selectivity to p-fluorobenzaldehyde 89%.

Example 20

The catalyst described in Run No. 2 of Example 12 is used in a 4 hour test with 4-phenylbenzoic acid at 433°C. The conversion is 92%, and the selectivity to p-phenylbenzaldehyde 94%.

Example 21

The catalyst described in Run No. 2 of Example 12 is used in a 4 hour test of trimethylacetic acid at 423°C. The conversion is 78%, and the selectivity to trimethylacetaldehyde 72%.

Example 22

The catalyst described in Run No. 2 of Example 12 is used in a 10 hour test with dimethylterephthalate at 435°C. The conversion is 89%, and the selectivity to terephthalalde-hyde 79%.

CLAIMS:

1. A process for the hydrogenation of a feedstock containing an aromatic or aliphatic carboxylic acid or an ester thereof to the corresponding aldehyde, characterised in that the process comprises reacting said carboxylic acid with a reducing gas at a temperature in the range of from 300° to 500°C and at a pressure of from 200 mm Hg to 100 atm. in the presence of a manganese dioxide catalyst supported on an activated alumina or an yttrium oxide catalyst which has been treated with copper, said latter catalyst comprising from 1 to 15 wt. % of copper and from 99 to 85 wt. % of yttrium, on an elemental basis.

2. A process according to claim 1 characterised in that the catalyst is manganese dioxide supported on gamma-alumina.

3. A process according to claim 1 or claim 2 characterised in that the catalyst comprises from 2 to 20 wt. % based on the weight of the support.

4. A process according to claim 1 characterised in that the catalyst is copper-treated yttrium oxide supported on alpha-alumina.

5. A process according to any of the preceding claims characterised in that the reactant and aldehyde produced therefrom are as follows:-

| | |
|---|---|
| benzoic acid or methyl benzoate | benzaldehyde |
| p-methylbenzoic acid or p-methylbenzoate | p-methylbenzaldehyde |
| p-tert-butylbenzoic acid | p-tert-butylbenzaldehyde |
| 3,5-dimethylbenzoic acid | 3,5-dimethylbenzaldehyde |
| methyl p-methoxybenzoate | p-methoxybenzaldehyde and anisole |
| p-fluorobenzoic acid | p-fluorobenzaldehyde |
| 4-phenylbenzoic acid | p-phenylbenzaldehyde |
| trimethylacetate | trimethylacetaldehyde |

dimethylterephthalate                    terephthalaldehyde

6.   A hydrogenation catalyst characterised in that it comprises from 2 to 20 wt. % of manganese dioxide, based on weight of support, supported on an activated alumina or from 1 to 15 wt. % of copper and from 99 to 85 wt. % of yttrium, on an elemental basis.

7.   A catalyst according to claim 6 characterised in that the catalyst is manganese dioxide supported on gamma-alumina.

8.   A catalyst according to claim 6 characterised in that the catalyst is from 5 to 8 wt. % of copper and from 95 to 92 wt. % of yttrium.

9.   A catalyst according to claim 6 characterised in that the catalyst is copper-treated yttrium oxide supported on alpha-alumina.

**European Patent Office**

## EUROPEAN SEARCH REPORT

| | DOCUMENTS CONSIDERED TO BE RELEVANT | | EP 85309528.9 |
|---|---|---|---|

| Category | Citation of document with indication, where appropriate, of relevant passages | Relevant to claim | CLASSIFICATION OF THE APPLICATION (Int. Cl.4) |
|---|---|---|---|
| X | DE - C - 710 746 (I.G. FARBENIN-DUSTRIE)<br>* Claim; examples * | 1 | C 07 C 45/41<br>C 07 C 47/52<br>C 07 C 47/02<br>B 01 J 23/34<br>B 01 J 23/76 |
| D,X | US - A - 4 328 373 (EDWIN J. STROJ-NY)<br>* Claims 1-7; column 3, line 42 * | 1,4,5 | |
| D,X | US - A - 4 093 661 (DAVID JOHN TRECKER et al.)<br>* Claims 1,7-9; column 2, line 57 *. | 1 | |
| X | DE - A - 2 050 061 (PRODUITS CHIMI-QUES PECHINEY)<br>* Claims * | 1,4,8, 9 | TECHNICAL FIELDS SEARCHED (Int. Cl.4) |
| A | US - A - 3 935 265 (ALLEN FEINSTEIN et al.)<br>* Claims; column 4, lines 20-27, 59 * | 1,2,5 | C 07 C 45/00<br>C 07 C 47/00<br>B 01 J |
| P,A | EP - A1 - O 150 961 (MITSUBISHI CHEMICAL INDUSTRIES)<br>* Claims 1,6,7 * | 1,5 | |
| A | EP - A1 - O 101 111 (SHELL INTERNA-TIONAL RESEARCH)<br>* Claims 1-7,17,18 * | 1,2,5 | |

The present search report has been drawn up for all claims

| Place of search | Date of completion of the search | Examiner |
|---|---|---|
| VIENNA | 11-04-1986 | REIF |

## EUROPEAN SEARCH REPORT

| | DOCUMENTS CONSIDERED TO BE RELEVANT | | EP 85309528.9 |
|---|---|---|---|

| Category | Citation of document with indication, where appropriate, of relevant passages | Relevant to claim | CLASSIFICATION OF THE APPLICATION (Int Cl 4) |
|---|---|---|---|
| D,A | US - A - 2 018 350 (OTTO DROSSBACH et al.) <br><br> * Claims 1,2 * <br><br> ---- | 1 | |
| | | | **TECHNICAL FIELDS SEARCHED (Int. Cl.4)** |
| The present search report has been drawn up for all claims | | | |

| Place of search | Date of completion of the search | Examiner |
|---|---|---|
| VIENNA | 11-04-1986 | REIF |